(19) Europäisches Patentamt

European Patent Office

Office européen des brevets

(11) **EP 1 462 121 A1**

(12) **EUROPEAN PATENT APPLICATION**
published in accordance with Art. 158(3) EPC

(43) Date of publication:
29.09.2004 Bulletin 2004/40

(51) Int Cl.7: **A61L 15/00**, A61F 13/02,
A61K 9/70, B32B 5/02,
B32B 27/36

(21) Application number: 02778009.7

(22) Date of filing: 30.10.2002

(86) International application number:
PCT/JP2002/011303

(87) International publication number:
WO 2003/037393 (08.05.2003 Gazette 2003/19)

(84) Designated Contracting States:
AT BE BG CH CY CZ DE DK EE ES FI FR GB GR
IE IT LI LU MC NL PT SE SK TR
Designated Extension States:
AL LT LV MK RO SI

(30) Priority: 31.10.2001 JP 2001335334

(71) Applicant: HISAMITSU PHARMACEUTICAL CO.,
INC.
Tosu-shi Saga 841-0017 (JP)

(72) Inventors:
• TATEISHI, T. Tsukuba Laboratory of HISAMITSU
Tsukuba-shi, Ibaraki 305-0856 (JP)
• HIGO, N. Tsukuba Laboratory of HISAMITSU
Tsukuba-shi, Ibaraki 305-0856 (JP)
• SATO, Shji
Kawasaki-shi Kanagawa 216-0003 (JP)

(74) Representative: Grünecker, Kinkeldey,
Stockmair & Schwanhäusser Anwaltssozietät
Maximilianstrasse 58
80538 München (DE)

(54) **PLASTER HAVING LAMINATED SUPPORT**

(57)    A patch comprising a laminated backing layer and a drug-containing pressure-sensitive adhesive layer,

wherein the laminated backing layer consists of a knitted fabric laminated on one side of a polyester film, and the drug-containing pressure-sensitive adhesive layer is laminated on the other side of the polyester film, wherein the knitted fabric has a 30% longitudinal modulus of 0.1 kg/5 cm to 20 kg/5 cm and a 30% lateral modulus of not more than 10 kg/5 cm, and a 30% longitudinal modulus/30% lateral modulus ratio of not less than 2.

## Description

### Technical Field

The present invention relates to a patch laminated with a drug-containing pressure-sensitive adhesive layer onto a laminated backing layer.

### Background Art

In the case of most pharmaceuticals, the drugs are orally administered in the form of tablets, capsules, syrups or the like. However, orally administered drugs are associated with certain drawbacks, such as high susceptibility to the first pass effect in the liver after their absorption or temporary blood levels in excess of requirement after administration, and the consequent side effects. Development of medicinal patches has been actively pursued with the goal of avoiding the drawbacks of oral administration. Patches not only compensate for these drawbacks but also offer benefits such as reduced administration frequency, better compliance and ease of both administration and its termination, and are particularly useful for elderly and infant patients.

The development of patches has led to increasingly stricter requirements for backing layers in recent years. In particular, patch backing layers is required to be flexible and exhibit no curling, and to have barrier property against drugs, i.e., drugs are not adsorbed onto the backing layers.

Research has therefore been carried out on various types of backing layers having such properties, and for example, Japanese Patent Application Laid-Open No. HEI 5-309128 discloses a patch comprising a pressure-sensitive adhesive layer laminated on the nonwoven fabric side of a backing layer obtained by laminating a polyester film with a thickness of 0.5-6 μm and a polyester nonwoven fabric with a basis weight of 5-20 g/m$^2$. Also, Japanese Patent Application Laid-Open No. HEI 8-104622 discloses a backing layer obtained by laminating a polymer film with a 50% modulus of 0.1-2.5 kg/5 cm, such as polyethylene or ethylene-vinyl acetate copolymer, with a nonwoven fabric or woven fabric having a 50% modulus of 0.1-1.5 kg/5 cm and a drape coefficient of 0.3-0.7. In addition, Japanese Patent No. 2505674 discloses a patch employing a solid fine particle-containing polyester film with a thickness of 0.5-4.9 μm and having a specific strength and ductility, wherein a backing layer sheet is laminated on the film.

However, patches employing such conventional backing layers have been inadequate from the viewpoint of flexibility, easily undergo curling, and are still lacking in terms of attachment ease, adhesion to skin and comfortability, while an additional disadvantage has been adsorption of drugs into the backing layers which impedes satisfactory permeation into the skin. No patch has yet been developed which satisfies all of these required properties.

### Disclosure of the Invention

The present invention has been accomplished in light of the aforementioned problems associated with the prior art, and its object is to provide a patch which has excellent flexibility, does not exhibit curling, is easy to attach and has excellent adhesion and comfortability, while also avoiding adsorption of the drug into the backing layer and thus ensuring adequate skin permeation of the drug.

As a result of much diligent research directed toward achieving the object stated above, the present inventors have discovered that by laminating a drug-containing pressure-sensitive adhesive layer on a laminated backing layer comprising a knitted fabric with specific properties laminated with a polyester film, it is possible to obtain a patch which is adequately resistant to curling and maintains a high level of flexibility, attachment ease, adhesion and comfortability while also satisfactorily preventing adsorption of the drug onto the backing layer. The present invention has been completed on the basis of this discovery.

Specifically, the patch of the invention is a patch comprising a laminated backing layer and a drug-containing pressure-sensitive adhesive layer, wherein the laminated backing layer consisting of a knitted fabric laminated on one side of a polyester film, and the drug-containing pressure-sensitive adhesive layer is laminated on the other side of the polyester film, wherein the knitted fabric has a 30% longitudinal modulus of 0.1 kg/5 cm to 20 kg/5 cm and a 30% lateral modulus of not more than 10 kg/5 cm, and a 30% longitudinal modulus/30% lateral modulus ratio of not less than 2.

Since the backing layer in the patch of the invention consists of a knitted fabric and a polyester film, and the 30% moduli of the knitted fabric in the different perpendicular directions (longitudinal and lateral directions) are in specific ranges and ratio, the flexibility is excellent and curling is adequately prevented even after lamination of the polyester film, while the patch having the laminated backing layer is also easy to attach to the body and the skin adhesion and comfortability are particularly excellent. In addition, since a drug-containing pressure-sensitive adhesive layer is laminated on the polyester film side of the laminated backing layer, drug adsorption onto the laminated backing layer is adequately prevented and skin permeation of the drug is therefore sufficient to produce a therapeutic effect.

**Best Mode for Carrying Out the Invention**

The patch of the invention comprises a laminated backing layer and a drug-containing pressure-sensitive adhesive layer, wherein the laminated backing layer consisting of a knitted fabric laminated on one side of a polyester film, and the drug-containing pressure-sensitive adhesive layer is laminated on the other side of the polyester film.

The laminated backing layer according to the invention will be explained first. The knitted fabric in the laminated backing layer of the invention has a cloth texture or structure wherein loops (stitches) are formed with yarn and the loops are alternately laced forward/backward and left/right, and thus it differs from woven fabrics or nonwoven fabrics. Because this type of knitted fabric has flexibility, elasticity and a soft feel, it is suitable as a patch backing layer. On the other hand, direct lamination of a drug-containing pressure-sensitive adhesive layer on a knitted fabric results in adsorption of the drug into the backing layer, and therefore according to the present invention, a polyester film with a high barrier property (drug barrier property) is situated between the knitted fabric and the pressure-sensitive adhesive layer to prevent adsorption of the drug into the backing layer and to achieve sufficient permeation of the drug into the skin for a therapeutic effect.

According to the invention, the knitted fabric used has a 30% longitudinal modulus of 0.1 kg/5 cm to 20 kg/5 cm and a 30% lateral modulus of not more than 10 kg/5 cm, and a 30% longitudinal modulus/30% lateral modulus ratio of not less than 2. By using this manner of knitted fabric it is possible to obtain a laminated backing layer having excellent flexibility and adequate resistance to curling even though laminated with the polyester film, while the patch provided with the laminated backing layer is easily attached to the human body and exhibits a high level of skin adhesion and comfortability.

Here, the longitudinal direction is the machine direction during the process of fabricating the knitted fabric, while the lateral direction is the direction perpendicular to the longitudinal direction, or in other words, the direction of width. The 30% longitudinal modulus of the knitted fabric of the invention is 0.1 kg/5 cm to 20 kg/5 cm, preferably 0.2 kg/5 cm to 15 kg/5 cm and more preferably 0.4 kg/5 cm to 12 kg/5 cm. If the 30% longitudinal modulus is less than 0.1 kg/5 cm, the knitted fabric is too soft and curling (or "warping") occurs in the longitudinal direction (machine direction) after lamination with the polyester film. On the other hand, a 30% longitudinal modulus of more than 20 kg/5 cm impairs the flexibility of the laminated backing layer and reduces the comfortability and skin adhesion of the obtained patch.

The 30% lateral modulus of the knitted fabric of the invention is not more than 10 kg/5 cm, preferably 0.1-8 kg/5 cm and more preferably 0.3-5 kg/5 cm. Since curling of the backing layer comprising the knitted fabric laminated with the polyester film tends to occur in the longitudinal direction, i.e. the machine direction during the process of lamination, a 30% lateral modulus of more than 10 kg/5 cm impairs the flexibility of the laminated backing layer and reduces the comfortability and skin adhesion of the obtained patch. On the other hand, a 30% lateral modulus below the aforementioned minimum results in a knitted fabric which is too soft and is more susceptible to curling after lamination with the polyester film.

According to the invention, even when using a knitted fabric with a 30% longitudinal modulus and 30% lateral modulus in the ranges specified above, if the 30% longitudinal modulus/30% lateral modulus ratio is less than 2, the softness in the laminating lateral direction during the process of laminating the knitted fabric and the polyester film (the direction of width during the process of lamination) becomes more influential, and curling also occurs in the lateral direction (width direction). The knitted fabric of the invention must have a 30% longitudinal modulus of 0.1 kg/5 cm to 20 kg/5 cm and a 30% lateral modulus of not more than 10 kg/5 cm, as well as a 30% longitudinal modulus/30% lateral modulus ratio of not less than 2 and preferably a 30% longitudinal modulus/30% lateral modulus ratio of not less than 3. There is no particular restriction on the upper limit of the 30% longitudinal modulus/30% lateral modulus ratio, but if the ratio is too large there will be a tendency toward slight deformation after cutting of the patch to the prescribed size, and therefore it is preferably not more than 20.

Throughout the present specification, "30% modulus" (30% longitudinal modulus or 30% lateral modulus) is the value measured according to the method of JIS L1018 (Cut strip method).

There are no particular restrictions on the material of the fibers composing the knitted fabric having the specific properties described above, but a polyester knitted fabric is preferred for better flexibility and elasticity, and from the standpoint of safety and general purpose use, a polyethylene terephthalate knitted fabric is especially preferred.

The basis weight of the knitted fabric is not particularly restricted, but if the basis weight is too low the handling properties will tend to be less than satisfactory, and if the basis weight is too high the flexibility and elasticity may be inadequate resulting in hardness; a range of 10-200 g/m$^2$ is therefore preferred.

There are also no restrictions on the thickness of the laminated backing layer consisting of the knitted fabric laminated on the polyester film, but if it is too thin the handling properties may be so poor as to diminish the ease of attachment, and if it is too thick the flexibility and elasticity may be so inadequate as to result in hardness, while also tending to promote fold-over during attachment; a range of 0.05-1 mm is therefore preferred.

According to the invention, the material of the polyester film laminated on the knitted fabric is not particularly restricted so long as it is a polyester, but a polyethylene terephthalate film is preferred from the standpoint of flexibility, safety and general purpose use. There are also no particular restrictions on the thickness of the polyester film, but if it is too thin it will be more susceptible to wrinkling when it is laminated during the process of lamination with the knitted fabric, and if it is too thick the flexibility will tend to be insufficient; a range of 0.5-12 µm is therefore preferred.

The polyester film of the invention has a longitudinal ductility of preferably at least 0.5% and less than 30%, and more preferably between 2% and 25%. With a longitudinal ductility of less than 0.5%, the flexibility of the laminated backing layer may be impaired and the comfortability and skin adhesion of the obtained patch will tend to be reduced. On the other hand, with a longitudinal ductility of not less than 30%, curling tends to occur more easily in the longitudinal direction.

The polyester film of the invention has a lateral ductility of preferably at not less than 2%, and more preferably not less than 4% and not more than 50%. With a lateral ductility of less than 2%, the flexibility of the laminated backing layer may be impaired and the comfortability and skin adhesion of the obtained patch will tend to be reduced. On the other hand, with a lateral ductility exceeding the aforementioned maximum, curling tends to occur more easily in the lateral direction.

Throughout the present specification, "ductility" (longitudinal ductility or lateral ductility) is the value measured in the manner described below, according to the tensile strength measuring method described in "Bandages" in the Japanese Pharmacopoeia. Specifically, a 12 mm-wide, 200 mm-long test piece is prepared, placed in a desiccator previously saturated with vapor of a saturated sodium nitrite solution, and allowed to stand for 4 hours at ordinary temperature. The gauge length is adjusted to 150 mm (length before pulling = 150 mm) with a pendulum tester, and both ends of the test piece are gripped with 25-50 mm wide anchors and pulled at a speed of 300 mm/min, after which the length at the time of cutting is measured. The ductility is calculated by the following formula based on the obtained measured value.

$$\text{Ductility [\%]} = (\{(\text{length at the time of cutting}) - (\text{length before pulling}))/(\text{length before pulling})\} \times 100$$

The laminated backing layer of the invention which consists of the aforementioned knitted fabric laminated on the polyester film has excellent flexibility, and its stiffness as an indicator of the flexibility is preferably not more than 30 mg and more preferably from 3 mg to 20 mg. If the stiffness is more than 30 mg, the flexibility of the laminated backing layer may be impaired and the comfortability and skin adhesion of the obtained patch will tend to be reduced. On the other hand, with a stiffness of less than the aforementioned minimum, the laminated backing layer tends to be too soft and the attachment ease tends to be reduced. Throughout the present specification, the "stiffness" is the value measured according to the method described in JIS L1096 (Gurley method).

The laminated backing layer of the invention preferably has the knitted fabric and polyester film adhesively laminated by an adhesive. There are no particular restrictions on the adhesive used, but it is preferably an adhesive comprising a hydrocarbon-based polymer and a tackifier, in order to result in satisfactory adhesion between the knitted fabric and polyester film. As hydrocarbon-based polymers there may be mentioned styrene-isoprene-styrene block copolymer, styrene-butadiene-styrene block copolymer, styrene-butadiene rubber, isoprene rubber, polyisobutylene and the like, but highly safe styrene-isoprene-styrene block copolymer is most preferred. As tackifiers there may be mentioned rosin derivatives (rosins, rosin glycerin esters, hydrogenated rosins, hydrogenated rosin glycerin esters, rosin pentaerythritol esters, etc.), alicyclic saturated hydrocarbon resins (ALCON P100 (Arakawa Chemical Co., Ltd.), etc.), aliphatic hydrocarbon resins (QUINTON B-170 (Nihon Zeon), etc.) terpene resins (YS resin PX-1150 (Yasuhara Chemical), etc.) and the like, and the tackifier content in the adhesive will generally be about 30-90 wt%.

For the patch of the invention, against the laminated backing layer consisting of a knitted fabric laminated on one side of the aforementioned polyester film, a drug-containing pressure-sensitive adhesive layer is further laminated on the other side of the polyester film. The composition of the drug-containing pressure-sensitive adhesive layer is not particularly restricted, and will normally contain suitable polymer materials, organic acids, absorption enhancers, plasticizers, tackifiers and the like in addition to the drug.

The types of drugs to be used for the invention are not particularly restricted so long as they are transdermally absorbed drugs, and as examples there may be mentioned hypnotics/sedatives (flurazepam hydrochloride, rilmazafone hydrochloride, phenobarbital, amobarbital, etc.), antipyretic antiphlogistic analgesics (butorphanol tartrate, perisoxal citrate, acetaminophen, mefenamic acid, diclofenac sodium, aspirin, alclofenac, ketoprofen, flurbiprofen, naproxen, piroxicam, pentazocine, indomethacin, glycol salicylate, aminopyrine, loxoprofen, etc.), steroidal antiinflammatory agents (hydrocortisone, prednisolone, dexamethasone, betamethasone), analeptic psychostimulants (methamphetamine hydrochloride, methylphenidate hydrochloride, etc.), psychoneural agents (imipran hydrochloride, diazepam, sertraline hydrochloride, fluvoxamine maleate, paroxetine hydrochloride, citalopram hydrobromide, fluoxetine hydrochloride, alprazolam, haloperidol, clomipramine, amitriptyline, desipramine, amoxapine, maprotiline, mianserin, setiptiline, trazadone, lofepramine, milnacipran, duloxetine, venlafexine, chlorpromazine hydrochloride, thioridazine, diazepam, meprobamate, etizolam, etc.), hormone agents (estradiol, estriol, progesterone, norethisterone acetate, methenolone acetate, testosterone, etc.), local anesthetics (lidocaine hydrochloride, procaine hydrochloride, tetracaine hydrochloride, dibucaine hydrochloride, propitocaine hydrochloride, etc.), urinary tract agents (oxybutynin hydrochloride, tamsulosin hydrochloride, propiverine hydrochloride, etc. ), skeletal muscle relaxants (tizanidine hydrochloride, eperisone hydrochloride, pridinol mesylate, suxamethonium hydrochloride, etc.), genital tract agents (ritodrine hydrochloride, meluadrine tartrate, etc.), antiepileptics (sodium valproate, clonazepam, carbamazepine, etc.), autonomic agents (carpronium chloride, neostigmine bromide, bethanechol chloride, etc.), antiparkinsonian agents (pergolide mesylate, bromocriptine mesylate, trihexyphenidyl hydrochloride, amantadine hydrochloride, ropinirole hydrochloride, talipexole hydrochloride, cabergoline, droxidopa, biperiden, selegiline hydrochloride, etc.), diuretics (hydroflumethiazide, furosemide, etc.), respiratory stimulants (loberine hydrochloride, dimorpholamine, naloxone hydrochloride, etc.), anti-migraine agents (dihydroergotamine mesylate, sumatriptan, ergotamine tartrate, flunarizine hydrochloride, cyproheptadine hydrochloride, etc.), antihistamines (clemastine fumarate, diphenhydramine tannate, chlorpheniramine maleate, diphenylpyraline hydrochloride, promethazine, etc.), bronchodilators (tulobuterol hydrochloride, procaterol hydrochloride, salbutamol sulfate, clenbuterol hydrochloride, fenoterol hydrobromide, terbutaline sulfate, isoprenaline sulfate, formoterol fumarate, etc.), cardiac stimulants (isoprenaline hydrochloride, dopamine hydrochloride), coronary vasodilators (diltiazem hydrochloride, verapamil hydrochloride, isosorbide dinitrate, nitroglycerin, nicorandil, etc.), peripheral vasodilators (nicametate citrate, tolazoline hydrochloride, etc.), stop smoking aids (nicotine, etc.), cardiovascular drugs (flunarizine hydrochloride, nicardipine hydrochloride, nitrendipine, nisoldipine, felodipine, amlodipine besylate, nifedipine, nilvadipine, manidipine hydrochloride, benidipine hydrochloride, enalapril maleate, temocapril hydrochloride, alacepril, imidapril hydrochloride, cilazapril, lisinopril, captopril, trandolapril, perindopril elbumin, atenolol, pindolol, bisoprolol fumarate, metoprolol tartrate, betaxolol hydrochloride, timolol maleate, bopindolol malonate, nipradilol, arotinolol hydrochloride, celiprolol hydrochloride, carvedilol, amosulalol hydrochloride, carteolol hydrochloride, bevantrol hydrochloride, terazosin hydrochloride, bunazosin hydrochloride, prazosin hydrochloride, doxazocine mesylate, valsartan, candesartan cilexetil, losartan potassium, clonidine hydrochloride, guanfacine hydrochloride, guanabenz acetate, etc.), antiarrhythmics (propranolol hydrochloride, alprenolol hydrochloride, procainamide hydrochloride, mexitilene hydrochloride, nadolol, disopyramide, etc.), anti-malignant ulcer agents (cyclophosphamide, fluorouracil, tegafur, procarbazine hydrochloride, ranimustine, irinotecan hydrochloride, fluridine, etc.), hypolipidemic drugs (pravastatin, simvastatin, bezafibrate, probucol, etc.), hypoglycemic agents (glibenclamide, chlorpropamide, tolbutamide, glymidine sodium, glybuzole, buformine hydrochloride, etc.), peptic ulcer agents (proglumide, cetraxate hydrochloride, spizofurone, cimetidine, glycopyrronium bromide, etc.), choleretic agents (ursodesoxycholic acid, osalmid, etc.), enterokinesis ameliorators (domperidone, cisapride, etc.), hepatic disease drugs (tiopronin, etc.), antiallergic agents (ketotifen fumarate, azelastine hydrochloride, etc.), antiviral agents (aciclovir, etc.), antivertigo agents (betahistine mesylate, difenidol hydrochloride, etc.), antibiotics (cefaloridine, cefdinir, cefpodoxime proxetil, cefaclor, clarithromycin, erythromycin, methylerythromycin, kanamycin sulfate, cycloserine, tetracycline, benzylpenicillin potassium, propicillin potassium, cloxacillin sodium, ampicillin sodium, bacampicillin hydrochloride, carbenicillin sodium, chloramphenicol, etc.), agents for addictive dependence (cyanamide, etc.), anorectic agents (mazindol, etc.), chemotherapeutics (isoniacide, ethionamide, pyrazinamide, etc.), blood clotting promoters (ticlopidine hydrochloride, warfarin potassium, etc.), anti-Alzheimer drugs (physostigmine, donepezil hydrochloride, tacrine, arecoline, xanomeline, etc.), serotonin receptor-antagonistic antiemetics (ondansetron hydrochloride, granisetron hydrochloride, ramosetron hydrochloride, azasetron hydrochloride, etc.), gout therapeutic agents (colchicine, probenecid, sulfinpyrazone, etc.), and narcotic analgesics (fentanyl citrate, morphine sulfate, morphine hydrochloride, codeine phosphate, cocaine hydrochloride, pethidine hydrochloride, etc.).

These drugs may be used alone or in combinations of two or more, and the drugs may be included in the form of inorganic salts, organic salts or free bases. Such drugs may be appropriately compounded, usually in the range of 0.1-60 wt% based on the weight of the total composition of the pressure-sensitive adhesive layer, taking into account adequate permeation of the patch, and reducing redness or other irritation of the skin.

Polymer materials which may be added to the pressure-sensitive adhesive layer in the patch of the invention are not particularly restricted, and there may be mentioned rubber-based polymers such as styrene-isoprene-styrene block copolymer, styrene-butadiene-styrene block copolymer, styrene-butadiene rubber and isoprene rubber, acrylic-based polymers, silicone-based polymers and the like, which may be used alone or in combinations of two or more.

Such polymer materials are appropriately added in a range of preferably 5-80 wt%, more preferably 10-70 wt% and most preferably 10-60 wt%, based on the weight of the total composition of the pressure-sensitive adhesive layer, taking into account formation of the pressure-sensitive adhesive layer and achieving adequate skin permeation of the drug.

An organic acid may also be added to the pressure-sensitive adhesive in the patch of the invention. As such organic acids there may be mentioned aliphatic (mono-, di-, tri-) carboxylic acids (acetic acid, propionic acid, isobutyric acid, caproic acid, caprylic acid, lactic acid, maleic acid, pyruvic acid, oxalic acid, succinic acid, tartaric acid, etc.),

aromatic carboxylic acids (phthalic acid, salicylic acid, benzoic acid, acetylsalicylic acid, etc.), alkylsulfonic acids (methanesulfonic acid, ethanesulfonic acid, propylsulfonic acid, butanesulfonic acid, polyoxyethylenealkylether sulfonic acid, etc.), alkylsulfonic acid derivatives (N-2-hydroxyethylpiperidine-N'-2-ethanesulfonic acid, etc.) and cholic acid derivatives (dehydrocholic acid, etc.), among which monocarboxylic acids or alkylsulfonic acids are preferred, and acetic acid is particularly preferred. These organic acids may be used as their salts, or as mixtures of the organic acids with their salts.

Such organic acids and/or their salts are suitably added in the range of preferably 0.01-20 wt%, more preferably 0.1-15 wt% and even more preferably 0.1-10 wt% based on the weight of the total composition of the pressure-sensitive adhesive layer, taking into account skin permeation of the drug and irritation of the skin. With a content of less than 0.01 wt%, the skin permeation of the drug will tend to be less than sufficient, and with a content of more than 20 wt%, skin irritation will tend to occur more readily.

The pressure-sensitive adhesive layer in the patch of the invention may also contain a absorption enhancer. The absorption enhancer may be any compound previously known to have absorption enhancing action into the skin, and as examples there may be mentioned:

(1) C6-20 fatty acids, aliphatic alcohols, fatty acid esters, fatty acid amides and fatty acid ethers (any of which may be saturated or unsaturated, or cyclic, straight-chain or branched),
(2) aromatic organic acids, aromatic alcohols and aromatic organic acid esters or ethers, and
(3) lactates, acetates, monoterpene-based compounds, sesquiterpene-based compounds, Azone, Azone derivatives, glycerin fatty acid esters, propyleneglycol fatty acid esters, sorbitan fatty acid esters (Span-type), polysorbates (Tween-type), polyethyleneglycol fatty acid esters, polyoxyethylene hydrogenated castor oils (HCO-based), polyoxyethylene alkyl ethers, sucrose fatty acid esters, vegetable oils, and the like.

Specifically, there are preferred caprylic acid, capric acid, caproic acid, lauric acid, myristic acid, palmitic acid, stearic acid, isostearic acid, oleic acid, linolic acid, linolenic acid, lauryl alcohol, myristyl alcohol, oleyl alcohol, isostearyl alcohol, cetyl alcohol, methyl laurate, hexyl laurate, diethanolamide laurate, isopropyl myristate, myristyl myristate, octyldodecyl myristate, cetyl palmitate, salicylic acid, methyl salicylate, ethylene glycol salicylate, cinnamic acid, methyl cinnamate, cresol, cetyl lactate, lauryl lactate, ethyl acetate, propyl acetate, geraniol, thymol, eugenol, terpineol, 1-menthol, borneolol, d-limonene, isoeugenol, isoborneol, nerol, dl-camphor, glycerin monocaprylate, glycerin monocaprate, glycerin monolaurate, glycerin monooleate, sorbitan monolaurate, sucrose monolaurate, polysorbate 20, propylene glycol, propylene glycol monolaurate, polyethylene glycol monolaurate, polyethylene glycol monostearate, polyoxyethylene lauryl ether, HCO-60, pyrothiodecane and olive oil, among which there are particularly preferred lauryl alcohol, myristyl alcohol, isostearyl alcohol, diethanolamide laurate, glycerin monocaprylate, glycerin monocaprate, glycerin monooleate, sorbitan monolaurate, propylene glycol monolaurate, polyoxyethylene lauryl ether and pyrothiodecane.

Such absorption enhancers may also be used in combinations of two or more, and are suitably added in the range of preferably 0.01-20 wt%, more preferably 0.05-10 wt% and even more preferably 0.1-5 wt%, based on the weight of the total composition of the pressure-sensitive adhesive layer, taking into account adequate permeability as a patch, and reducing irritation of the skin which may include redness, edema or the like.

A plasticizer may also be added to the pressure-sensitive adhesive layer in the patch of the invention. As such plasticizers there may be mentioned petroleum-based oils (paraffin-based processed oils, naphthene-based processed oils, aromatic-based processed oils, etc.), squalane, squalene, vegetable oils (olive oil, camellia oil, castor oil, tall oil, peanut oil, etc.), silicone oil, dibasic acid esters (dibutyl phthalate, dioctyl phthalate, etc.), liquid rubbers (polybutene, liquid isoprene rubber, etc.), liquid fatty esters (isopropyl myristate, hexyl laurate, diethyl sebacate, diisopropyl sebacate, etc.), diethylene glycol, polyethylene glycol, glycol salicylate, propylene glycol, dipropylene glycol, triacetin, triethyl citrate and crotamiton. Particularly preferred among these are liquid paraffin, liquid polybutene, crotamiton, isopropyl myristate, diethyl sebacate and hexyl laurate.

Such plasticizers may also be used in combinations of two or more, and the content of such plasticizers based on the total composition of the pressure-sensitive adhesive layer is preferably 5-70 wt%, more preferably 10-60 wt% and even more preferably 10-50 wt%, taking into account adequate permeability and maintaining sufficient cohesive force as a patch.

A tackifier may further be added to the pressure-sensitive adhesive layer in the patch of the invention. As such tackifiers there may be mentioned rosin derivatives (rosins, rosin glycerin esters, hydrogenated rosins, hydrogenated rosin glycerin esters, rosin pentaerythritol esters, etc.), alicyclic saturated hydrocarbon resins (ALCON P100 (Arakawa Chemical Co., Ltd.), etc.), aliphatic hydrocarbon resins (QUINTON B-170 (Nihon Zeon), etc.) terpene resins (KURIALON P-125 (Yasuhara Chemical), etc.), maleic acid resins, and the like. Particularly preferred among these are hydrogenated rosin glycerin esters, alicyclic saturated hydrocarbon resins, aliphatic hydrocarbon resins and terpene resins.

The tackifier content based on the total composition of the pressure-sensitive adhesive layer is in the range of preferably 5-70 wt%, more preferably 5-60 wt% and even more preferably 10-50 wt%, taking into account adequate adhesive force as a patch and reducing irritation of the skin during peeling.

If necessary, the pressure-sensitive adhesive layer in the patch of the invention may also contain other added antioxidants, fillers, crosslinking agents, preservatives, ultraviolet absorbers and the like. As antioxidants there are preferred tocopherols and their ester derivatives, ascorbic acid, ascorbic stearic acid esters, nordihydroguaiaretic acid, dibutylhydroxytoluene (BHT) and butylhydroxyanisole. As fillers there are preferred calcium carbonate, magnesium carbonate, silicates (for example, aluminum silicate and magnesium silicate), silicic acid, barium sulfate, calcium sulfate, calcium zincate, zinc oxide and titanium oxide. As crosslinking agents there are preferred thermosetting resins such as amino resins, phenol resins, epoxy resins, alkyd resins and unsaturated polyesters, isocyanate compounds and block isocyanate compounds, organic crosslinking agents, and inorganic crosslinking agents such as metals and metal compounds. As preservatives there are preferred ethyl paraoxybenzoate, propyl paraoxybenzoate and butyl paraoxybenzoate. As ultraviolet absorbers there are preferred p-aminobenzoic acid derivatives, anthranilic acid derivatives, salicylic acid derivatives, coumarin derivatives, amino acid-based compounds, imidazoline derivatives, pyrimidine derivatives and dioxane derivatives.

Such antioxidants, fillers, crosslinking agents, preservatives and ultraviolet absorbers are suitably added in the range of preferably not more than 10 wt%, more preferably not more than 5 wt% and even more preferably not more than 2 wt% based on the weight of the total composition of the pressure-sensitive adhesive layer of the patch.

The pressure-sensitive adhesive layer having the composition described above may be fabricated by any of the following methods. Specifically, the drug-containing base component may be heated to melting and coated onto the release sheet or backing layer, and then attached onto the backing layer or release sheet. Alternatively, the drug-containing base component may be dissolved in a solvent such as toluene, hexane or ethyl acetate, spread onto the release sheet or backing layer and dried for removal of the solvent, and then attached to the backing layer or release sheet to give a patch.

The patch of the invention is sufficient if it is a patch comprising a drug-containing pressure-sensitive adhesive layer laminated on the polyester film side of a laminated backing layer consisting of the aforementioned knitted fabric laminated on the polyester film, and there are no additional restrictions on the composition, construction or various component materials, or on their types.

The pressure-sensitive adhesive layer may also be provided with a release sheet layer, and for example, there may be used release sheets selected from films of silicon-treated polyesters (polyethylene terephthalate, etc.), polyvinyl chloride and polyvinylidene chloride, laminated films of woodfree paper and polyolefins, and the like, laminated on the contact side of the pressure-sensitive adhesive layer.

(Examples)

The present invention will now be explained in greater detail based on examples and comparative examples, with the understanding that the invention is not limited to the examples, and various modifications may be incorporated which are not outside of the technical scope of the invention. Throughout the examples and comparative examples, the entire "%" refers to "wt%", unless otherwise specified.

Example 1

(Fabrication of a backing layer)

A polyethylene terephthalate knitted fabric with a 30% longitudinal modulus of 8.5 kg/5 cm, a 30% lateral modulus of 2.7 kg/5 cm (30% longitudinal modulus/30% lateral modulus ratio of 3.14) and a basis weight of 120 g/m$^2$ was bonded to a polyethylene terephthalate film with a thickness of 6 $\mu$m, a longitudinal ductility of 12.5% and a lateral ductility of 20.6% using an adhesive comprising styrene-isoprene-styrene block copolymer (CALIFLEX TR1107, product of Shell Chemical) and a terpene-based resin (YS resin PX-1150, Yasuhara Chemical) to fabricate a laminated backing layer. The thickness of the obtained laminated backing layer was 0.55 mm, and the stiffness was 18.5 mg.

(Fabrication of a patch)

Procaterol hydrochloride, lauryl alcohol, sodium acetate and liquid paraffin were measured into a mortar and thoroughly mixed, and the obtained mixture was added to a mixed solution consisting of styrene-isoprene-styrene block copolymer, an acrylic-based polymer (DURO-TAK87-4098, National Starch & Chemicals), an alicyclic saturated hydrocarbon resin (ALCON P100, Arakawa Chemical Co., Ltd.), ethyl acetate and toluene, to prepare a coating solution having the following composition (without solvent):

| Styrene-isoprene-styrene block copolymer | 18.0% |
| Acrylic-based polymer | 22.0% |
| Alicyclic saturated hydrocarbon resin | 29.5% |
| Liquid paraffin | 19.5% |
| Lauryl alcohol | 3.0% |
| Sodium acetate | 3.0% |
| Procaterol hydrochloride | 5.0% |

The obtained coating solution was then coated onto a polyethylene terephthalate release film and the solvent was removed by drying to form a pressure-sensitive adhesive layer (thickness: 75 µm). The pressure-sensitive adhesive layer was spread onto the exposed side of the polyethylene terephthalate film of the previously fabricated laminated backing layer to give a patch.

Comparative Example 1

A patch was obtained in the same manner as Example 1, except that the pressure-sensitive adhesive layer was spread onto the exposed side of the polyethylene terephthalate knitted fabric of the laminated backing layer.

Comparative Example 2

A patch was obtained in the same manner as Example 1, except that a polyethylene film (thickness: 50 µm) was used instead of the polyethylene terephthalate film.

Comparative Example 3

A patch was obtained in the same manner as Example 1, except that a backing layer composed only of a polyethylene terephthalate knitted fabric was used instead of the laminated backing layer.

Example 2

Fentanyl citrate, pyrothiodecane, sodium acetate and liquid paraffin were measured into a mortar and thoroughly mixed, and the obtained mixture was added to a mixed solution consisting of styrene-isoprene-styrene block copolymer, polyisobutylene, an alicyclic saturated hydrocarbon resin (ALCON P100, product of Arakawa Chemical Co., Ltd.) and toluene, to prepare a coating solution having the following composition (without solvent):

| Styrene-isoprene-styrene block copolymer | 16.5% |
| Polyisobutylene | 11.0% |
| Alicyclic saturated hydrocarbon resin | 34.5% |
| Liquid paraffin | 29.5% |
| Pyrothiodecane | 3.0% |
| Sodium acetate | 1.5% |
| Fentanyl citrate | 4.0% |

A patch was obtained in the same manner as Example 1, except for the use of this coating solution.

Comparative Example 4

A patch was obtained in the same manner as Example 2, except that the pressure-sensitive adhesive layer was spread onto the exposed side of the polyethylene terephthalate knitted fabric of the laminated backing layer.

Comparative Example 5

A patch was obtained in the same manner as Example 2, except that a soft vinyl chloride film (thickness: 100 µm) was used instead of the polyethylene terephthalate film.

Comparative Example 6

A patch was obtained in the same manner as Example 2, except that a backing layer composed only of a polyethylene terephthalate knitted fabric was used instead of the laminated backing layer.

Hairless mouse drug skin permeation test

Dorsal skin of hairless mice was peeled off and fixed in a flow-through cell (5 cm$^2$) having peripherally circulating 37°C warm water, with the dermal side as the receptor layer side. The patches obtained in Examples 1 and 2 and Comparative Examples 1-6 were attached to the horny layer side and, using physiological saline as the receptor layer, sampling was carried out at speed of 5 ml/hr every 2 hours for a period of 24 hours. For the receptor solutions obtained at each interval, the flow rate was precisely measured, the drug concentration was measured by high-performance liquid chromatography, the drug permeation rate per hour was calculated and the drug skin permeation rate per unit area in a stationary state was determined. Samples with large permeation rates were judged as having excellent percutaneous absorbency. The results are shown in Table 1.

Table 1

|  | Drug skin permeation rate ($\mu$g/m$^2$/hr) |
|---|---|
| Example 1 | 3.4 |
| Comparative Example 1 | 1.1 |
| Comparative Example 2 | 1.6 |
| Comparative Example 3 | 1.0 |
| Example 2 | 24.1 |
| Comparative Example 4 | 11.2 |
| Comparative Example 5 | 7.5 |
| Comparative Example 6 | 10.8 |

As clearly seen by the results in Table 1, the patches obtained in Examples 1 and 2 according to the present invention were confirmed to have notably improved drug skin permeation rates compared to the patches obtained in Comparative Examples 1-6 which were outside the scope of the invention, given the same pressure-sensitive adhesive layer composition.

Examples 3-10 and Comparative Examples 7-12

(Fabrication of backing layers)

Polyethylene terephthalate knitted fabrics having the 30% longitudinal moduli, 30% lateral moduli and basis weights shown in Tables 2 and 3 were bonded to polyethylene terephthalate films having the longitudinal ductilities, lateral ductilities and thicknesses shown in Tables 2 and 3 using an adhesive comprising styrene-isoprene-styrene block copolymer (CALIFLEX TR1107, Shell Chemical) and a terpene-based resin (YS resin PX-1150, Yasuhara Chemical) to fabricate laminated backing layers. The thicknesses and stiffnesses of the obtained laminated backing layers are shown in Tables 2 and 3.

(Fabrication of patches)

Ketoprofen, 1-menthol and liquid paraffin were measured into a mortar and thoroughly mixed, and the obtained mixture was added to a mixed solution comprising styrene-isoprene-styrene block copolymer, polyisobutylene, a hydrogenated rosin ester (KE-311, product of Arakawa Chemical Co., Ltd.) and toluene, to prepare a coating solution having the following composition (without solvent):

| Styrene-isoprene-styrene block copolymer | 6.5% |
|---|---|
| Polyisobutylene | 24.5% |
| Hydrogenated rosin ester | 36.8% |

(continued)

| Liquid paraffin | 31.5% |
| 1-Menthol | 0.5% |
| Ketoprofen | 0.2% |

The obtained coating solution was then coated onto a polyethylene terephthalate release film and the solvent was removed by drying to form a pressure-sensitive adhesive layer (thickness: 120 µm). The pressure-sensitive adhesive layer was spread onto the exposed side of the polyethylene terephthalate film of the previously fabricated laminated backing layer to obtain a patch.

Example 11

A patch was obtained in the same manner as Example 3, except that a urethane-based adhesive (DIABOND DA3042M, Nogawa Chemical Co., Ltd.) was used instead of the adhesive comprising the styrene-isoprene-styrene block copolymer and terpene-based resin.

Comparative Example 3

A patch was obtained in the same manner as Example 3, except that laminated backing layers composed only of polyethylene terephthalate films having the longitudinal ductilities, lateral ductilities and thicknesses shown in Tables 2 and 3 were used instead of the laminated backing layer described above.

Organoleptic tests

The patches fabricated in Examples 3-11 and Comparative Examples 7-13 were each cut to a 20 $cm^2$ area and used for the organoleptic tests described below after attachment for 8 hours onto the arms of ten volunteers. The results of the organoleptic tests are shown in Tables 2 and 3.

(Curling)

The patch was peeled from the release film and the degree of curling was evaluated based on the following scale.

　　1: High degree of curling
　　2: Moderate curling
　　3: No curling

(Attachment ease)

The patch was attached onto the skin and the attachment ease was evaluated based on the following scale.

　　1: Difficult to attach
　　2: Somewhat difficult to attach but acceptable
　　3: Easy to attach

(Comfortability)

The patch was attached to the skin and the comfortability was evaluated based on the following scale.

　　1: Lacking flexibility, uncomfortable
　　2: Somewhat lacking flexibility and slightly uncomfortable, but acceptable
　　3: Excellent flexibility, no discomfort

(Adhesive strength)

The patch was attached to the skin and the adhesion was evaluated based on the following scale.

1: Falling off
2: Unacceptable folding
3: Partial folding, but acceptable
4: Absolutely no peeling

(Cohesion between fabric and film)

The degree of cohesion between the knitted fabric and film of the patch was evaluated based on the following scale.

1: Peeling between fabric and film
2: Satisfactory cohesion between fabric and film, with no interlayer peeling

(Overall evaluation)

An overall evaluation was assigned from each of the organoleptic test evaluations, based on the following scale.

A: Very good
B: Good
C: Somewhat poor
D: Poor

Table 2

| | | | Example | | | | | | | | |
|---|---|---|---|---|---|---|---|---|---|---|---|
| | | | 3 | 4 | 5 | 6 | 7 | 8 | 9 | 10 | 11 |
| Knitted fabric | 30% Longitudinal modulus (kg/5 cm) | | 7.1 | 13.6 | 16.9 | 4.7 | 0.9 | 0.9 | 0.9 | 0.9 | 7.1 |
| | 30% Lateral modulus (kg/5 cm) | | 1.6 | 5.2 | 6.8 | 0.9 | 0.4 | 0.4 | 0.4 | 0.4 | 1.6 |
| | 30% Longitudinal modulus/30% Lateral modulus | | 4.44 | 2.62 | 2.49 | 5.22 | 2.25 | 2.25 | 2.25 | 2.25 | 4.44 |
| | Basis weight (g/m$^2$) | | 89 | 178 | 35 | 122 | 95 | 95 | 95 | 95 | 90 |
| Polyethylene terephthalate film | Longitudinal ductility (%) | | 12.5 | 22.1 | 28.1 | 7.2 | 16.7 | 0.05 | 33.6 | 3.3 | 12.5 |
| | Lateral ductility (%) | | 20.6 | 13.4 | 45.3 | 9.9 | 14.2 | 4.2 | 49.3 | 1.6 | 20.6 |
| | Thickness ($\mu$m) | | 5 | 5 | 10 | 2 | 6 | 1.5 | 12 | 2 | 5 |
| Stiffness (mg) | | | 14.0 | 22.1 | 19.0 | 12.3 | 13.1 | 3.9 | 29.0 | 7.7 | 14.7 |
| Backing layer thickness (mm) | | | 0.32 | 0.81 | 0.18 | 0.57 | 0.36 | 0.35 | 0.37 | 0.35 | 0.32 |
| Curling | | | 3 | 3 | 3 | 3 | 3 | 3 | 2 | 3 | 3 |
| Attachment ease | | | 3 | 3 | 3 | 3 | 3 | 3 | 3 | 3 | 3 |
| Comfortability | | | 3 | 2 | 2 | 3 | 3 | 2 | 3 | 2 | 3 |
| Adhesion | | | 4 | 4 | 4 | 4 | 4 | 3 | 4 | 3 | 4 |
| Fabric/film cohesion | | | 2 | 2 | 2 | 2 | 2 | 2 | 2 | 2 | 1 |
| Overall evaluation | | | A | B | B | A | A | B | B | B | B |

# EP 1 462 121 A1

Table 3

| | | Comparative Example | | | | | | |
|---|---|---|---|---|---|---|---|---|
| | | 7 | 8 | 9 | 10 | 11 | 12 | 13 |
| Knitted fabric | 30% Longitudinal modulus (kg/5 cm) | 0.03 | 27.2 | 17.4 | 6.2 | 3.4 | 18.3 | |
| | 30% Lateral modulus (kg/5 cm) | 0.02 | 9.2 | 12.5 | 7.6 | 0.9 | 7.5 | |
| | 30% Longitudinal modulus/30% Lateral modulus | 1.50 | 2.96 | 1.39 | 0.82 | 3.78 | 2.44 | |
| | Basis weight (g/m$^2$) | 77 | 106 | 101 | 82 | 6 | 241 | |
| Polyethylene terephthalate film | Longitudinal ductility (%) | 12.5 | 12.5 | 38.5 | 22.1 | 16.7 | 0.05 | 16.7 |
| | Lateral ductility (%) | 20.6 | 20.6 | 40.2 | 13.4 | 14.2 | 4.2 | 14.2 |
| | Thickness ($\mu$m) | 5 | 5 | 5 | 5 | 6 | 1.5 | 6 |
| Stiffinces (mg) | | 9.2 | 34.2 | 28.6 | 16.4 | 9.7 | 28.8 | 8.3 |
| Backing layer thickness (mm) | | 0.29 | 0.38 | 0.38 | 0.30 | 0.03 | 1.29 | 0.006 |
| Curling | | 1 | 3 | 1 | 1 | 3 | 3 | 3 |
| Attachment ease | | 1 | 3 | 3 | 1 | 1 | 3 | 1 |
| Comfortability | | 3 | 1 | 1 | 2 | 2 | 1 | 1 |
| Adhesion | | 2 | 1 | 1 | 2 | 3 | 2 | 2 |
| Fabric/film cohesion | | 2 | 2 | 2 | 2 | 2 | 2 | - |
| Overall evaluation | | D | D | D | D | C | C | D |

As clearly seen by the results of organoleptic tests in Tables 2 and 3, the patches obtained in Examples 3-11 according to the present invention were confirmed to maintain at satisfactory levels with a good balance, the occurrence of curling, the ease of attachment, the comfortability, the adhesion strength and the cohesion between fabrics and films compared to the patches obtained in Comparative Examples 7-13 which were outside the scope of the invention.

**Industrial Applicability**

According to the present invention, as explained above, it is possible to obtain a patch which has excellent flexibility, exhibits no curling and uses a backing layer which does not adsorb the drug. Consequently, it is easy to attach and has excellent adhesion and comfortability, while the lack of adsorption of the drug into the backing layer also ensures adequate skin permeation of the drug.

**Claims**

1.  A patch comprising a laminated backing layer and a drug-containing pressure-sensitive adhesive layer, wherein the laminated backing layer consists of a knitted fabric laminated on one side of a polyester film, and the drug-containing pressure-sensitive adhesive layer is laminated on the other side of the polyester film, wherein the knitted fabric has a 30% longitudinal modulus of 0.1 kg/5 cm to 20 kg/5 cm and a 30% lateral modulus of not more than 10 kg/5 cm, and a 30% longitudinal modulus/30% lateral modulus ratio of not less than 2.

2.  A patch according to claim 1, wherein the polyester film has a longitudinal ductility of not less than 0.5% and less than 30%.

3.  A patch according to claim 1, wherein the polyester film has a lateral ductility of not less than 2% and a thickness of 0.5-12 $\mu$m.

4. A patch according to claim 1, wherein the stiffness of the laminated backing layer is not more than 30 mg.

5. A patch according to claim 1, wherein the basis weight of the knitted fabric is 10 g/m$^2$ to 200 g/m$^2$ and the thickness of the laminated backing layer is 0.05-1 mm.

6. A patch according to claim 1, wherein the knitted fabric is a polyester knitted fabric.

7. A patch according to claim 1, wherein the knitted fabric and the polyester film are glued together by an adhesive comprising a hydrocarbon-based polymer and a tackifier.

8. A patch according to claim 7, wherein said hydrocarbon-based polymer is styrene-isoprene-styrene block copolymer.

## INTERNATIONAL SEARCH REPORT

| International application No. |
|---|
| PCT/JP02/11303 |

**A. CLASSIFICATION OF SUBJECT MATTER**
Int.Cl⁷  A61L15/00, A61F13/02, A61K9/70, B32B5/02, 27/36

According to International Patent Classification (IPC) or to both national classification and IPC

**B. FIELDS SEARCHED**

Minimum documentation searched (classification system followed by classification symbols)
Int.Cl⁷  A61L15/00-15/02, A61F13/02, A61K9/70, B32B5/02, 27/36

Documentation searched other than minimum documentation to the extent that such documents are included in the fields searched

Electronic data base consulted during the international search (name of data base and, where practicable, search terms used)
WPI/L

**C. DOCUMENTS CONSIDERED TO BE RELEVANT**

| Category* | Citation of document, with indication, where appropriate, of the relevant passages | Relevant to claim No. |
|---|---|---|
| Y | JP 60-75428 A  (Sekisui Chemical Co., Ltd.), 27 April, 1985 (27.04.85), Claims; page 3, lower left column, lines 9 to 13; example 3 (Family: none) | 1-8 |
| Y | JP 5-84260 A  (Bando Chemical Industries Ltd.), 06 April, 1993 (06.04.93), Claims 1, 4; Par. No. [0003] (Family: none) | 1-8 |
| A | JP 2001-29383 A  (Nitto Denko Corp.), 06 February, 2001 (06.02.01), Claims (Family: none) | 1-8 |

☐ Further documents are listed in the continuation of Box C.   ☐ See patent family annex.

| | |
|---|---|
| *    Special categories of cited documents:<br>"A"   document defining the general state of the art which is not considered to be of particular relevance<br>"E"   earlier document but published on or after the international filing date<br>"L"   document which may throw doubts on priority claim(s) or which is cited to establish the publication date of another citation or other special reason (as specified)<br>"O"   document referring to an oral disclosure, use, exhibition or other means<br>"P"   document published prior to the international filing date but later than the priority date claimed | "T"   later document published after the international filing date or priority date and not in conflict with the application but cited to understand the principle or theory underlying the invention<br>"X"   document of particular relevance; the claimed invention cannot be considered novel or cannot be considered to involve an inventive step when the document is taken alone<br>"Y"   document of particular relevance; the claimed invention cannot be considered to involve an inventive step when the document is combined with one or more other such documents, such combination being obvious to a person skilled in the art<br>"&"   document member of the same patent family |

| Date of the actual completion of the international search | Date of mailing of the international search report |
|---|---|
| 03 February, 2003 (03.02.03) | 18 February, 2003 (18.02.03) |

| Name and mailing address of the ISA/<br>   Japanese Patent Office | Authorized officer |
|---|---|
| Facsimile No. | Telephone No. |

Form PCT/ISA/210 (second sheet) (July 1998)

14